# EUROPEAN PATENT SPECIFICATION

(11) **EP 2 568 834 B1**
(45) Date of publication and mention of the grant of the patent: **26.02.2020**
(21) Application number: 11781219.8
(22) Date of filing: 11.05.2011
(51) Int. Cl.: A41D 1/00, A61B 5/024, A41D 27/20, A61B 5/11

(54) **GLOBAL POSITIONING SYSTEM GARMENT**
GPS-KLEIDUNGSSTÜCK
VÊTEMENT POUR SYSTÈME DE POSITIONNEMENT GLOBAL

(30) Priority: 11.05.2010 US 777788
(43) Date of publication of application: 20.03.2013
(73) Proprietor: NIKE Innovate C.V., Beaverton, OR 97005-6453 (US)
(72) Inventor: SOKOLOWSKI, Susan, L., Beaverton, OR 97005-6453 (US)
(74) Representative: Beattie, Alex Thomas Stewart
(86) International application number: PCT/US2011/036105
(87) International publication number: WO 2011/143334

(56) References cited:
- EP-A1- 2 177 156
- WO-A1-2009/089569
- US-A- 5 210 881
- US-A1- 2007 139 875
- US-A1- 2007 226 871
- US-A1- 2007 241 887
- US-A1- 2007 299 325
- US-A1- 2008 125 288
- US-A1- 2008 125 288

## Description

### SUMMARY OF THE INVENTION

Embodiments of the invention are defined by the claims below, not this summary. A high-level overview of various aspects of the invention are provided here for that reason, to provide an overview of the disclosure, and to introduce a selection of concepts that are further described below in the detailed-description section below. This summary is not intended to identify key features or essential features of the claimed subject matter, nor is it intended to be used as an aid in isolation to determine the scope of the claimed subject matter.

Global Positioning System (GPS) receivers use measurements from satellites to determine the position of the GPS receiver. GPS receivers typically determine their position by computing time delays between transmission and reception of signals transmitted from satellites and received by the receiver on or near the surface of the earth. The time delays are utilized to calculate the distance from the receiver to each of the satellites that are in view of the receiver. GPS receivers require a line of sight to the satellites in order to obtain a signal representative of the true distance from the satellite to the receiver. Therefore, any object in the path of the signal has the potential to interfere with the reception of that signal.

Handheld GPS receivers are often used during outdoor activities like camping, hiking and geocaching. In addition to route navigation, cyclists, runners, skiers and other athletes may utilize GPS devices during training or competitions to record training parameters like speed, distance and altitude. Despite their usefulness during sporting activities, it is often cumbersome to carry a GPS device during training and competition without interfering with athletic performance. Moreover, during high intensity sporting activities it is very difficult to hold a handheld GPS device in a position to receive a good satellite signal. As the use of GPS technology in conjunction with outdoor sporting activities increases, the need for a conveniently attaching a GPS receiver to an athlete while minimizing GPS signal blockage has arisen.

US 2007/139875 discloses a body conforming, reusable, washable, textile holder for removably holding at least one electronic device in close bodily contact. The holder may be a wrap or tube constructed to enclose a portion of the body where the electronic device is to be held. The electronic device may be a cellular telephone, a wireless communication device, a personal audio device, a wearable computer, an electronic monitoring device, an electronic identification card, or other electronic device. The invention also relates to a system comprising the body conforming holder and an electronic device for use therewith, and a method for holding such an electronic device in close bodily contact by wearing the holder.

Embodiments of the present invention provide systems and methods according to the claims.

### BRIEF DESCRIPTION OF THE DRAWING

Illustrative embodiments of the present invention are described in detail below with reference to the attached drawing figures, wherein:
FIG. 1 depicts a front view of a garment embodying features of the present invention;
FIG. 2 depicts a rear view of a garment embodying features of the present invention;
FIG. 3 depicts a side view of a garment;
FIG. 4 depicts a rear view of another garment;
FIG. 5 depicts an athlete training utilizing a GPS signal optimizing garment;
FIG. 6 depicts a block diagram of an overall method of athletic training utilizing a GPS signal optimizing garment; and
FIG. 7 depicts a front view of heart rate monitor tabs that may be used in a garment in accordance with the present invention.

### DETAILED DESCRIPTION OF THE INVENTION

The subject matter of the present invention is described with specificity herein to meet statutory requirements. However, the description itself is not intended to necessarily limit the scope of claims. Rather, the claimed subject matter might be embodied in other ways to include different steps or combinations of steps similar to the ones described in this document, in conjunction with other present or future technologies. Although the terms "step" and/or "block" or "module" etc. might be used herein to connote different components of methods or systems employed, the terms should not be interpreted as implying any particular order among or between various steps herein disclosed unless and except when the order of individual steps is explicitly described.

The present invention relates to a garment for carrying a GPS device during athletic training or competition to optimize the GPS signal without interfering with athletic activities. The invention further relates to methods for performing athletic training using a GPS signal optimizing garment. A garment in accordance with the present invention may have a three-dimensional pocket so as to hold the GPS receiver between the shoulders of an athlete training while wearing the garment positioned to promote a GPS receiver obtaining an optimal GPS signal.

Accordingly, in one aspect, the present invention provides a Global Positioning System (GPS) signal optimizing garment. The garment comprises a shirt that extends around at least a portion of a wearer's torso in an as worn position, the shirt having a front and a back. The garment provides a three-dimensional pocket attached to the back of the shirt at least 2.5cm (one inch) from a neckline of the back of the shirt, the pocket having a size to hold a GPS device. The garment further comprises four heart rate monitor tabs attached to the inside of the garment below sleeves thereof, wherein two heart rate monitor tabs are below each sleeve to receive and retain heart rate monitor chest straps and enable a heart rate monitor device to be attached to the chest of the wearer when the garment is in as worn position.

In yet another aspect, the present invention provides a method for performing athletic training utilizing a GPS signal optimizing garment. The method includes providing a garment according to the above, powering on a GPS device, inserting the GPS device in the pocket of the garment, and wearing the garment during athletic training and/or athletic competition.

GPS signal optimizing generally refers to enabling a GPS receiver to rapidly acquire the strongest GPS satellite signal, thereby increasing the accuracy of the receiver. GPS devices rely on the data received through the radio signals sent by a network of satellites orbiting the earth that send data regarding the altitude, longitude, and latitude of the GPS receiver. The satellite signals are sent with enough strength to reach the Earth's surface, however any object in the path of the signal has the potential to interfere with the reception of that signal. As such, factors like narrow streets and high buildings, wipers running back and forth across a windshield, tree canopies, mountains, and other large objects, even highway overpasses, can interfere with or interrupt the GPS signal. Similarly, the upper body of an athlete may interfere with GPS signal reception when the athlete carries a GPS device in his/her hand or in a pant or short pocket. Also, movement on an extremity can be a problem for tracking athletic performance. For example, GPS watches are generally considerably heavier than traditional watches and may significantly affect the training of high performance athletes. GPS watches are also inherently less accurate than GPS receivers attached to the core of the wearer's body for measuring parameters such as the distance travelled. Having to carry a GPS receiver in his/her hand can also interference with athletic training or competition. For example, during a high intensity activity such as running, holding a bulky handheld GPS device could significantly affect the speed and distance travelled of an athlete. Accordingly, the GPS signal reception of a receiver used by an athlete may be optimized by positioning the GPS receiver on the athlete's body so that an unobstructed line of sight from the receiver to the GPS satellites is provided. In addition, a GPS signal optimizing garment should prevent interference with the movement of the wearer of the garment.

Embodiments of the present invention provide GPS signal optimizing garments, and methods for performing athletic training using a GPS signal optimizing garment. Having briefly described an overview of embodiments of the present invention, an exemplary GPS signal optimizing garment is described below.

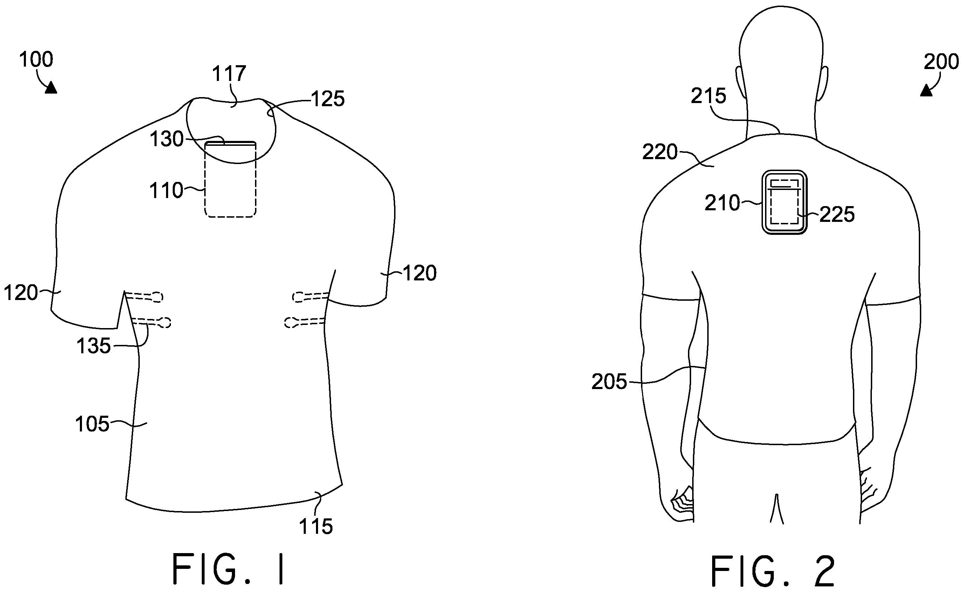

Referring to the drawings in general and FIGS. 1-5 in particular, an exemplary GPS signal optimizing garment is depicted in various views. Embodiments of the invention comprise a shirt. Further, the depictions in the drawings are for exemplary purposes only and are in no way meant to limit the scope of the present invention to any type of outdoor activity or athletic training technique. Further the materials to create the GPS devices, as well as the material properties of GPS devices are well known in the art and will not be discussed in further detail herein.

Referring now to FIG. 1, a front view of a garment embodying features of the present invention is illustrated and designated generally as reference numeral 100. Garment 100 includes a shirt 105, a three-dimensional pocket 110, with the shirt 105 including a front 115, back 117, sleeves 120, a neckline 125 and an opening 130. Garment 100 can be any type of material for use in athletic activity. For example, garment 100 may be constructed of form fitting material including but not limited to woven and non-woven knits, spandex, lycra, and athletic mesh. Shirt 105 extends around at least a portion of a wearer's torso, with the front 115 covering the wearer's chest and back 117 covering the wearer's back. Shirt 105 can include sleeves 120 as shown; however shirt 105 can be long-sleeved or sleeveless. Shirt 105 also includes a neckline 125 that extends around a portion of a wearer's neck. Attached to the back 117 of shirt 105 is a three-dimensional pocket 110. Pocket 110 has a size to securely hold a GPS device. The back 117 of shirt 105 may include an opening 130 at the inner top edge of pocket 110. Opening 130 may allow insertion of a GPS device (not shown) into pocket 110. For optimal GPS signal reception by the GPS device, pocket 110 is attached to the back 117 of shirt 105 at least 2.5cm (one inch) from neckline 125. In embodiments, pocket 110 may be located within 7.6cm (three inches) from the neckline 125 of shirt 105. Garment 100 also includes heart rate monitor tabs 135 for attaching a heart rate monitor device. Generally, heart rate monitor tabs may be shaped or sized for a particular type or brand of heart rate monitor device. In embodiments, heart rate monitor tabs 135 may be 'universal' tabs designed to facilitate receiving and retaining various types of known heart rate monitor device. The heart rate monitor tabs 135 are attached to the inside of garment 100 below sleeves 120. Heart rate monitor tabs 135 enable a heart rate monitor device to be conveniently and comfortably attached to the chest of the wearer when 100 is in an as worn position. Garment 100 includes multiple heart rate monitor tabs 135 to ensure an optimal fit of a heart rate monitor device during athletic training and/or competition.

Turning now to FIG. 2, a rear view of a GPS signal optimizing garment embodying features of the present invention is illustrated and designated generally as reference number 200. Garment 200 includes a shirt 205, a three-dimensional pocket 210, a neckline 215, a back portion 220 of shirt 205 and a GPS device 225. The placement of pocket 210 on garment 200 may increase the ability of GPS device 225 to receive signals. In embodiments the pocket 210 may be located between the shoulder blades of the back of a wearer in an as worn position. GPS device 225 may include GPS devices known in the art. In one example, pocket 210 may be constructed to hold a GPS device 225 with dimensions of at least 5.1cm (2 inches) by 10.2cm (4 inches) by 2.5cm (1 inch). In embodiments, GPS device 225 may include internal communication transmitters, receivers and processors.

Pocket 210 may be constructed of collapsible material and/or of yieldable fabric including but not limited to spacer mesh. Pocket 210 may be fabricated by cutting and sewing a patch of collapsible material into a three-dimensional shape and attaching the edges of the pocket 210 to the back of shirt 205. Pocket 210 may be placed on shirt 205 by stitching, gluing or any other technique.

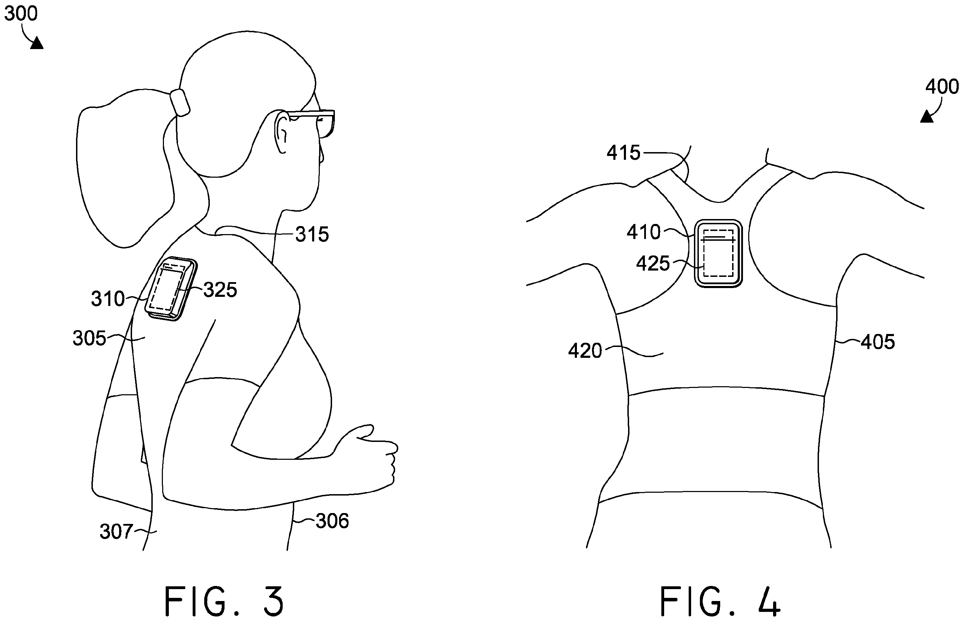

Turning now to FIG. 3, a side view of a GPS signal optimizing garment is illustrated and designated generally as reference number 300. Garment 300 includes a shirt 305 that extends around a portion of a wearer's torso having a front 306, a back 307, a neckline 315. Attached to the back 307 of shirt 305 is a three-dimensional pocket 310. Pocket 310 may have a size to securely hold a GPS device. For optimal GPS signal reception by the GPS device, pocket 310 may be attached to the back 307 of shirt 305 at least 2.5cm (one inch) from neckline 315. The placement of pocket 310 may allow a wearer of garment 300 to transport a GPS device without having to hold the GPS device. Rather than uncomfortably carrying a GPS device in his/her hand, a wearer of garment 300 can transport a GPS device in pocket 310. As such, garment 300 may reduce GPS signal interference and limit interference with the athletic performance of the wearer.

Turning now to FIG. 4, a rear view of a another GPS signal optimizing garment is illustrated and designated generally as reference number 400. Garment 400 includes a bra 405, a three-dimensional pocket 410, a neckline 415, a back portion 420 of bra 405 and a GPS device 425. Three-dimensional pocket 410 may be attached to the back 420 of bra 405 2.5cm (one inch) from the neckline 415. The placement of pocket 410 on garment 400 may increase the ability of GPS device 425 to receive signals. The pocket 410 may be located between the shoulder blades of the back of a wearer in an as worn position. GPS device 425 may include GPS devices known in the art. In embodiments, GPS device 425 may include internal communication transmitters, receivers and processors. GPS device 425 may also include an accelerometer for measuring acceleration forces. Data obtained from the accelerometer may be used in conjunction with the initial position and velocity from the GPS device to continuously determine the location and velocity of the device. This data may be use to provide location information of the GPS device that is independent of GPS data and may be particularly important in instances when the GPS device is unable to clearly receive GPS signals.

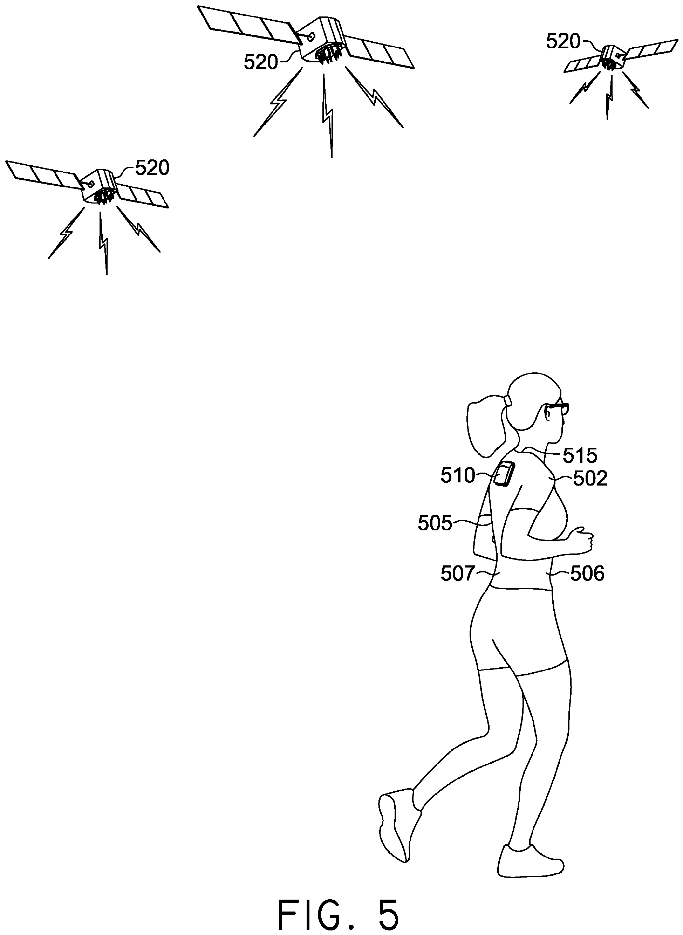

Turning now to FIG. 5, a diagram of an athlete training utilizing a GPS signal optimizing garment is illustrated and designated generally as reference number 500. The training method includes a GPS signal optimizing garment 502 having a shirt 505 that extends around a portion of a wearer's torso. The shirt 505 may have a front 506, a back 507 and a neckline 515. Attached to the back 507 of shirt 505 is a three-dimensional pocket 510. Pocket 510 may have a size to securely hold a GPS device (not shown). The GPS device may receive radio signals from GPS satellites 520 and utilize the data received through the radio signals to determine parameters including the altitude, longitude, and/or latitude of the GPS receiver. Knowing these parameters may enable an athlete to determine their location, and record training parameters including but not limited to speed, altitude and distance. For optimal GPS signal reception by the GPS device, pocket 510 may be attached to the back 507 of shirt 305 at least 2.5cm (one inch) from neckline 515. The placement of pocket 510 may allow a wearer of garment 502 to train with a GPS device without having to hold the GPS device. Rather than uncomfortably carrying a GPS device in his/her hand that interferes with training or competition, an athlete wearing garment 502 can transport a GPS device in pocket 510. As such, garment 502 may reduce GPS signal interference and limit interference with the athletic performance of the wearer. The three-dimensional nature of pocket 510 also enables an athlete to securely wear a GPS device without experiencing the discomfort associated with having the GPS device in a two-dimensional pocket. While the diagram illustrates running, embodiments of the present invention may be used in other sporting activities where GPS signal optimization in conjunction with unencumbered hands would be useful. By way of example and not limitation, the present invention may be utilized in sporting activities including, skiing, rowing, and cycling.

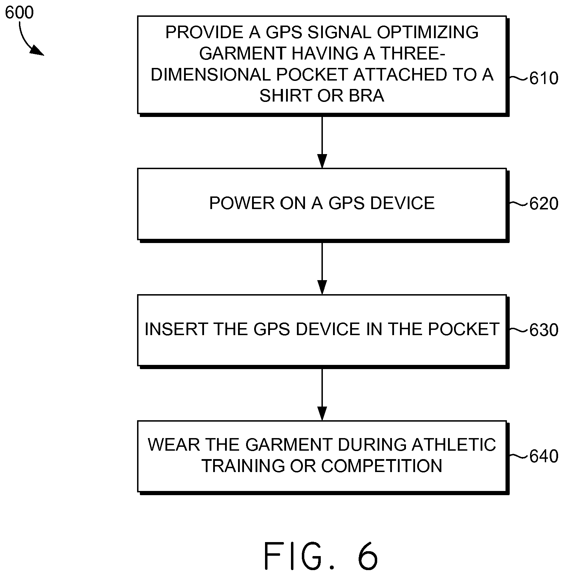

Referring now to FIG. 6, a block diagram is provided that illustrates a method 500 of athletic training utilizing a GPS signal optimizing garment. Initially, as shown at block 510, a GPS signal optimizing garment may be provided. The garment may be a shirt or sport bra that extends around at least a portion of a wearer's torso having a front and a back. The garment may provide a three-dimensional pocket attached to the back of the shirt at least 2.5cm (one inch) from the neckline of the shirt, the pocket having a size to hold a GPS device. A GPS device may be powered on, as shown at block 620. The GPS device may be inserted in the pocket of the garment, as shown at block 630. The garment, containing the GPS device removably secured in the three-dimensional pocket, may be worn during athletic training or competition, as shown at block 640.

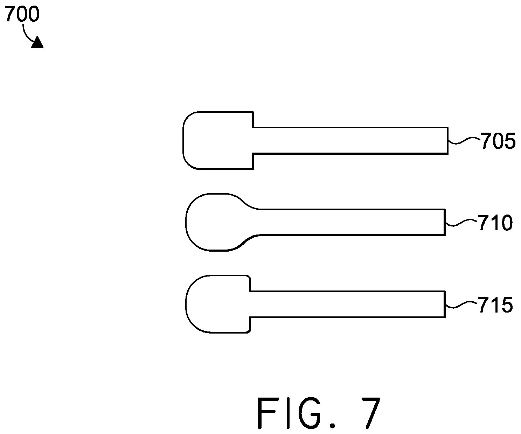

Turning now to FIG. 7, a front view of heart rate monitor tabs that may be used in a garment in accordance with the present invention are illustrated and designated generally as reference numeral 700. Heart rate monitor tabs 705 and 715 are illustrative examples of heart rate monitor tabs shaped or sized for a particular type or brand of heart rate monitor device. By way of example only, heart rate monitor tabs shaped like tab 705 may be used to receive and retain SUUNTO brand heart rate monitor chest straps, while heart rate monitor tabs shaped like tab 715 may be used for POLAR brand heart rate monitor chest straps. Heart rate monitor tab 710 is a further illustrative example of a universal heart rate monitor tab designed to facilitate retaining various types of known heart rate monitor devices. Heart rate monitor tabs 705, 710, and/or 715 may be affixed to the GPS optimizing garments of present invention.

Embodiments of the present invention have been described with the intent to be illustrative rather than restrictive. Alternative embodiments will become apparent to those skilled in the art that do not depart from its scope. A skilled artisan may develop alternative means of implementing the aforementioned improvements without departing from the scope of the present invention.

## Claims

1. A Global Positioning System (GPS) signal optimizing garment (100, 200, 300, 400, 502), the garment (100, 200, 300, 400, 502) comprising:
a shirt (105, 205, 305, 505) that extends around at least a portion of a wearer's torso in an as worn position, the shirt (105, 205, 305, 505) having a front (115, 306, 506) and a back (117, 220, 307, 420, 507); and a three-dimensional pocket (110, 210, 310, 410, 510) attached to the back (117, 220, 307, 420, 507) of the shirt (105, 205, 305, 505) at least 2.5cm (one inch) from a neckline (125, 215, 315, 415, 515) of the back (117, 220, 307, 420, 507) of the shirt (105, 205, 305, 505), the pocket (110, 210, 310, 410, 510) having a size to hold a GPS device (225, 325, 425),
**characterised in that** the garment (100, 200, 300, 400, 502) further comprises four heart rate monitor tabs (135, 700) attached to the inside of the garment (100, 200, 300, 400, 502) below sleeves (120) thereof, wherein two heart rate monitor tabs (135, 700) are below each sleeve (120) to receive and retain heart rate monitor chest straps and enable a heart rate monitor device to be attached to the chest of the wearer when the garment (100, 200, 300, 400, 502) is in an as worn position.

2. The garment (100, 200, 300, 400, 502) of claim 1, wherein the pocket (110, 210, 310, 410, 510) is located within 7.6cm (three inches) from the neckline (125, 215, 315, 415, 515) of the back (117, 220, 307, 420, 507) of the shirt (105, 205, 305, 505).

3. The garment (100, 200, 300, 400, 502) of claim 2, wherein the pocket (110, 210, 310, 410, 510) is constructed of collapsible material, and wherein a patch of the collapsible material for the pocket (110, 210, 310, 410, 510) is cut and sewn into a three-dimensional shape and attached at its edges to the shirt(105, 205, 305, 505).

4. The garment (100, 200, 300, 400, 502) of claim 1, wherein the garment (100, 200, 300, 400, 502) is constructed of form fitting, athletic material.

5. The garment (100, 200, 300, 400, 502) of claim 4, wherein the pocket (110, 210, 310, 410, 510) is fabricated from yieldable fabric.

6. The garment (100, 200, 300, 400, 502) of claim 5, wherein the pocket (110, 210, 310, 410, 510) is fabricated from spacer mesh.

7. The garment (100, 200, 300, 400, 502) of claim 6, wherein the pocket (110, 210, 310, 410, 510) is constructed to hold a GPS device (225, 325, 425) measuring at least 5cm (2 inches) by 10.1cm (4 inches) by 2.5cm (1 inch).

8. The garment (100, 200, 300, 400, 502) of claim 6, wherein the pocket (110, 210, 310, 410, 510) is located between the shoulder blades of the back of the wearer in an as worn position.

9. A system for transporting a Global Positioning System (GPS) device (225, 325, 425), the system comprising a GPS signal optimizing garment (100, 200, 300, 400, 502) according to any preceding claim, the GPS device (225, 325, 425) removably secured in the pocket (110, 210, 310, 410, 510).

10. The system of claim 9, wherein the GPS device (225, 325, 425) includes an internal communication transmitter, and internal communication receiver and a GPS processor.

11. A method for performing athletic training utilizing a Global Positioning System (GPS) signal optimizing garment (100, 200, 300, 400, 502), the method comprising:
providing a garment (100, 200, 300, 400, 502) according to claim 1;
powering on a GPS device (225, 325, 425);
inserting the GPS device (225, 325, 425) in the pocket (110, 210, 310, 410, 510) of the garment (100, 200, 300, 400, 502); and
wearing the garment (100, 200, 300, 400, 502) during at least one of athletic training and athletic competition.

12. The method of claim 11, wherein the pocket (110, 210, 310, 410, 510 is fabricated from yieldable fabric and wherein the pocket (110, 210, 310, 410, 510) is located between the shoulder blades of the back of the wearer in an as worn position.

## Patentansprüche

1. GPS-(globales Positionierungssystem)-Signaloptimierungskleidungsstück (100, 200, 300, 400, 502), wobei das Kleidungsstück (100, 200, 300, 400, 502) Folgendes umfasst:
ein Hemd (105, 205, 305, 505), das im getragenen Zustand wenigstens einen Teil des Oberkörpers eines Trägers umgibt, wobei das Hemd (105, 205, 305, 505) eine Vorderseite (115, 306, 506) und eine Rückseite (117, 220, 307, 420, 507) und eine dreidimensionale Tasche (110, 210, 310, 410, 510) hat, die auf der Rückseite (117, 220, 307, 420, 507) des Hemds (105, 205, 305, 505) wenigstens 2,5 cm (ein Zoll) von einem Ausschnitt (125, 215, 315, 415, 515) der Rückseite (117, 220, 307, 420, 507) des Hemds (105, 205, 305, 505) befestigt ist, wobei die Tasche (110, 210, 310, 410, 510) eine Größe zum Aufnehmen eines GPS-Geräts (225, 325, 425) hat,
**dadurch gekennzeichnet, dass** das Kleidungsstück (100, 200, 300, 400, 502) vier Herzfrequenzmonitorlaschen (135, 700) umfasst, die an der Innenseite des Kleidungsstücks (100, 200, 300, 400, 502) unterhalb der Ärmel (120) davon angebracht sind, wobei sich zwei Herzfrequenzmonitorlaschen (135, 700) unter jedem Ärmel (120) befinden, um Herzfrequenzmonitor-Brustbänder aufzunehmen und zu halten und um es zu ermöglichen, einen Herzfrequenzmonitor an der Brust des Trägers anzubringen, wenn das Kleidungsstück (100, 200, 300, 400, 502) im getragenen Zustand ist.

2. Kleidungsstück (100, 200, 300, 400, 502) nach Anspruch 1, wobei sich die Tasche (110, 210, 310, 410, 510) innerhalb von 7,6 cm (drei Zoll) vom Ausschnitt (125, 215, 315, 415, 515) der Rückseite (117, 220, 307, 420, 507) des Hemds (105, 205, 305, 505) befindet.

3. Kleidungsstück (100, 200, 300, 400, 502) nach Anspruch 2, wobei die Tasche (110, 210, 310, 410, 510) aus faltbarem Material konstruiert ist und wobei ein Aufnäher aus dem faltbaren Material für die Tasche (110, 210, 310, 410, 510) ausgeschnitten und zu einer dreidimensionalen Form genäht und mit seinen Rändern am Hemd (105, 205, 305, 505) angebracht wird.

4. Kleidungsstück (100, 200, 300, 400, 502) nach Anspruch 1, wobei das Kleidungsstück (100, 200, 300, 400, 502) aus formschlüssigem Athletikmaterial hergestellt ist.

5. Kleidungsstück (100, 200, 300, 400, 502) nach Anspruch 4, wobei die Tasche (110, 210, 310, 410, 510) aus elastischem Stoff hergestellt ist.

6. Kleidungsstück (100, 200, 300, 400, 502) nach Anspruch 5, wobei die Tasche (110, 210, 310, 410, 510) aus Abstandsgewirke hergestellt ist.

7. Kleidungsstück (100, 200, 300, 400, 502) nach Anspruch 6, wobei die Tasche (110, 210, 310, 410, 510) so konstruiert ist, dass sie ein GPS-Gerät (225, 325, 425) mit Maßen von wenigstens 5 cm (2 Zoll) mal 10,1 cm (4 Zoll) mal 2,5 cm (1 Zoll) aufnehmen kann.

8. Kleidungsstück (100, 200, 300, 400, 502) nach Anspruch 6, wobei sich die Tasche (110, 210, 310, 410, 510) im getragenen Zustand zwischen den Schulterblättern auf dem Rücken des Trägers befindet.

9. System zum Transportieren eines GPS-(Global Positioning System)-Geräts (225, 325, 425), wobei das System ein GPS-Signaloptimierungskleidungsstück (100, 200, 300, 400, 502) nach einem vorherigen Anspruch umfasst, wobei das GPS-Gerät (225, 325, 425) entfernbar in der Tasche (110, 210, 310, 410, 510) befestigt ist.

10. System nach Anspruch 9, wobei das GPS-Gerät (225, 325, 425) einen internen Kommunikationssender, einen internen Kommunikationsempfänger und einen GPS-Prozessor beinhaltet.

11. Verfahren zum Durchführen von Athletiktraining mit einem GPS-(Global Positioning System)-Signaloptimierungskleidungsstück (100, 200, 300, 400, 502), wobei das Verfahren Folgendes beinhaltet:
Bereitstellen eines Kleidungsstücks (100, 200, 300, 400, 502) nach Anspruch 1;
Einschalten eines GPS-Geräts (225, 325, 425);
Einstecken des GPS-Geräts (225, 325, 425) in die Tasche (110, 210, 310, 410, 510) des Kleidungsstücks (100, 200, 300, 400, 502); und
Tragen des Kleidungsstücks (100, 200, 300, 400, 502) bei Athletiktraining und/oder einem Athletikwettbewerb.

12. Verfahren nach Anspruch 11, wobei die Tasche (110, 210, 310, 410, 510) aus einem elastischen Stoff gefertigt ist und wobei sich die Tasche (110, 210, 310, 410, 510) im getragenen Zustand zwischen den Schulterblättern auf dem Rücken des Trägers befindet.

## Revendications

1. Vêtement d'optimisation de signal d'un système de positionnement global (GPS) (100, 200, 300, 400, 502), le vêtement (100, 200, 300, 400, 502) comprenant :
une chemise (105, 205, 305, 505) qui s'étend autour d'au moins une partie du torse d'une personne qui le porte en position portée, la chemise (105, 205, 305, 505) ayant un devant (115, 306, 506) et un dos (117, 220, 307, 420, 507) ; et
une poche en trois dimensions (110, 210, 310, 410, 510) fixée sur le dos (117, 220, 307, 420, 507) de la chemise (105, 205, 305, 505) à au moins 2,5 cm (un pouce) de l'encolure (125, 215, 315, 415, 515) du dos (117, 220, 307, 420, 507) de la chemise (105, 205, 305, 505), la poche (110, 210, 310, 410, 510) ayant une dimension permettant de contenir un dispositif GPS (225, 325, 425),
**caractérisé en ce que** le vêtement (100, 200, 300, 400, 502) comprend en outre quatre languettes de dispositif de surveillance de fréquence cardiaque (135, 700) fixées à l'intérieur du vêtement (100, 200, 300, 400, 502) au-dessous des manches (120) de celui-ci, dans lequel deux languettes de dispositif de surveillance de fréquence cardiaque (135, 700) sont au-dessous de chaque manche (120) pour recevoir et retenir des sangles de poitrine du dispositif de surveillance de fréquence cardiaque et permettre de fixer un dispositif de surveillance de fréquence cardiaque à la poitrine de la personne qui le porte quand le vêtement (100, 200, 300, 400, 502) est en position portée.

2. Vêtement (100, 200, 300, 400, 502) selon la revendication 1, dans lequel la poche (110, 210, 310, 410, 510) est située à moins de 7,6 cm (trois pouces) de l'encolure (125, 215, 315, 415, 515) du dos (117, 220, 307, 420, 507) de la chemise (105, 205, 305, 505).

3. Vêtement (100, 200, 300, 400, 502) selon la revendication 2, dans lequel la poche (110, 210, 310, 410, 510) est construite de matériau compressible, et dans lequel une pièce du matériau compressible de la poche (110, 210, 310, 410, 510) est coupée et cousue en une forme en trois dimensions et fixée sur ses bords à la chemise (105, 205, 305, 505).

4. Vêtement (100, 200, 300, 400, 502) selon la revendication 1, dans lequel le vêtement (100, 200, 300, 400, 502) est construit dans un matériau sportif, épousant les formes.

5. Vêtement (100, 200, 300, 400, 502) selon la revendication 4, dans lequel la poche (110, 210, 310, 410, 510) est fabriquée à partir d'un tissu déformable.

6. Vêtement (100, 200, 300, 400, 502) selon la revendication 5, dans lequel la poche (110, 210, 310, 410, 510) est fabriquée à partir d'un tissu filet.

7. Vêtement (100, 200, 300, 400, 502) selon la revendication 6, dans lequel la poche (110, 210, 310, 410, 510) est construite pour contenir un dispositif GPS (225, 325, 425) mesurant au moins 5 cm (2 pouces) sur 10,1 cm (4 pouces) sur 2,5 cm (1 pouce).

8. Vêtement (100, 200, 300, 400, 502) selon la revendication 6, dans lequel la poche (110, 210, 310, 410, 510) est située entre les omoplates du dos de la personne qui le porte en position portée.

9. Vêtement permettant de transporter un dispositif de système de positionnement global (GPS) (225, 325, 425), le système comprenant un vêtement d'optimisation de signal GPS (100, 200, 300, 400, 502) selon l'une quelconque des revendications précédentes, le dispositif GPS (225, 325, 425) étant assujetti de manière amovible dans la poche (110, 210, 310, 410, 510).

10. Vêtement selon la revendication 9, dans lequel le dispositif GPS (225, 325, 425) inclut un émetteur de communication interne et un récepteur de communication interne et un processeur GPS.

11. Procédé permettant de pratiquer un entraînement sportif en utilisant un vêtement d'optimisation de signal d'un système de positionnement global (GPS) (100, 200, 300, 400, 502), le procédé consistant à :
fournir un vêtement (100, 200, 300, 400, 502) selon la revendication 1 ;
mettre sous tension un dispositif GPS (225, 325, 425) ;
introduire le dispositif GPS (225, 325, 425) dans la poche (110, 210, 310, 410, 510) du vêtement (100, 200, 300, 400, 502) ; et
porter le vêtement (100, 200, 300, 400, 502) pendant soit un entraînement sportif, soit une compétition sportive.

12. Procédé selon la revendication 11, dans lequel la poche (110, 210, 310, 410, 510 est fabriquée à partir d'un tissu déformable et dans lequel la poche (110, 210, 310, 410, 510) est située entre les omoplates du dos de la personne qui le porte en position portée.
